(19) Europäisches Patentamt
European Patent Office
Office européen des brevets

(11) Veröffentlichungsnummer: **0 000 560**
**B1**

(12) **EUROPÄISCHE PATENTSCHRIFT**

(45) Veröffentlichungstag der Patentschrift: **03.02.82**

(21) Anmeldenummer: **78100464.3**

(22) Anmeldetag: **21.07.78**

(51) Int. Cl.³: **C 07 B 19/02**, C 12 P 7/42, C 07 C 59/01, C 07 D 213/55

(54) **Verfahren zur Herstellung von optisch aktiven alpha-Hydroxycarbonsäuren.**

(30) Priorität: **26.07.77 CH 9218/77**

(43) Veröffentlichungstag der Anmeldung:
**07.02.79 Patentblatt 79/3**

(45) Bekanntmachung des Hinweises auf die Patenterteilung:
**03.02.82 Patentblatt 82/5**

(84) Benannte Vertragsstaaten:
**BE CH DE FR GB NL**

(56) Entgegenhaltungen:
**CHEMICAL ABSTRACTS, vol. 47, 1953 3366i**
**I.U.P.A.C. & I.U.B. "Enzyme Nomenclature", 1972, Elsevier, Amsterdam, Seite 46**

(73) Patentinhaber: **F. HOFFMANN-LA ROCHE & CO. Aktiengesellschaft**
**CH-4002 Basel (CH)**

(72) Erfinder: **Suhara, Yasuji**
**9-506 Dream Height 1403-banchi Matano-cho Totsuha-ku Yokohama-shi Kanagawa-Ken (JP)**
Erfinder: **Maruyama, Hiromi**
**2231-77-banchi Kamigo-cho Totsuka-ku Yokohama-shi Kanagawa-ken (JP)**
Erfinder: **Sawada, Toyoaki**
**Zengyo danchi 5-7-203 fujisawa 3768-3 Fujisawa-shi Kanagawa-ken (JP)**
Erfinder: **Ogawa, Mayumi**
**1459-banchi Dai Kamakura-shi Kanagawa-ken (JP)**
Erfinder: **Yokose, Kazuteru**
**1. 480-banchi, Horie Urayasu-machi Higashitkatsushikagun Chiba-ken (JP)**
Erfinder: **Watanabe, Kimihiro**
**2009-59, Kamitsuchidana Ayase-machi Koza-gun Kanagawa-ken (JP)**
Erfinder: **Fujiu Morio**
**12-18, Ikego 3-chome, ZUshi-shi Kanagawa-ken (JP)**

(74) Vertreter: **Wuesthoff, Franz, Dr.-Ing. et al,**
**Patentanwälte Wuesthoff -v. Pechmann-Behrens-Goetz Schweigerstrasse 2**
**D-8000 München 90 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 560 B1

# Verfahren zur Herstellung von optisch aktiven α-Hydroxycarbonsäuren

Die vorliegende Erfindung betrifft ein neues Verfahren zur Herstellung von optisch aktiven α-Hydoxycarbonsäuren, insbesondere ein Verfahren zur Herstellung von optisch reinen D- oder L-α-Hydroxycarbonsäuren.

Die Herstellung von optisch aktiven α-Hydroxycarbonsäuren wurde bisher durch Racematspaltung von D,L-α-Hydroxycarbonsäuren mit Hilfsmitteln, wie optisch aktiven Aminen, durchgeführt. Dieses bekannte Verfahren hat sich jedoch als unwirtschaftlich erwiesen, insbesondere weil die Hilfsmittel kostspielig sind und die Auftrennung kompliziert ist.

Nach dem erfindungsgemässen Verfahren ist es nun möglich, optisch reine α-Hydroxycarbonsäuren mit hoher Ausbeute in sehr einfacher Weise zu erhalten. Dieses Verfahren ist dadurch gekennzeichnet, dass man eine D,L-α-Hydroxycarbonsäure der allgemeinen Formel

$$\underset{\displaystyle OH}{R{-}\underset{|}{C}H{-}COOH} \qquad I$$

worin R einen verzweigten oder geradkettigen Alkylrest mit 1—13 C-Atomen oder einen Pyridylrest darstellt,

oder ein Salz davon mittels enantiospezifische Dehydrogenaseaktivität aufweisenden, zu den Genera Streptomyces, Pseudomonas oder Bacillus oder zur Coryneformgruppe gehörenden Mikroorganismen asymmetrisch zur α-Ketocarbonsäure bzw. zu einem Salz davon dehydriert, aus einem erhaltenen Salz die Säure freisetzt und schliesslich das nicht dehydrierte Enantiomere der α-Hydroxycarbonsäure und gegebenenfalls die α-Ketocarbonsäure aus dem Reaktionsmedium isoliert.

Gemäss einer bevorzugten Ausführungsform des erfindungsgemässen Verfahrens wird die erhaltene α-Ketocarbonsäure in an sich bekannter Weise zur α-Hydroxycarbonsäure reduziert und diese als Ausgangsmaterial in das Verfahren zurückgeführt.

Typische Beispiele von racemischen α-Hydroxycarbonsäuren der Formel I sind 2-Hydroxypropionsäure, 2-Hydroxybuttersäure, 2-Hydroxyvaleriansäure, 2-Hydroxy-3-methylbuttersäure, 2-Hydroxycapronsäure, 2-Hydroxy-4-methylvaleriansäure, 2-Hydroxy-3,3-dimethylbuttersäure, 2-Hydroxyheptylsäure, 2-Hydroxy-4-methylcapronsäure, 2-Hydroxycaprylsäure, 2-Hydroxypelargonsäure, 2-Hydroxydecansäure, 2-Hydroxyundecansäure, 2-Hydroxydodecansäure, 2-Hydroxytridecansäure, 2-Hydroxytetradecansäure, 2-Hydroxypentadecansäure, α-Hydroxy-3-pyridylessigsäure und dgl.

Die im erfindungsgemässen Verfahren verwendbaren Mikroorganismen umfassen die zu den Genera Streptomyces, Pseudomona oder Bacillus oder zur Coryneformgruppe gehörenden, enantiospezifische Dehydraseaktivität aufweisenden Mikroorganismen. Im Rahmen der vorliegenden Erfindung bedeutet der Ausdruck "enantiospezifische Dehydraseaktivität die Fähigkeit, eines der Enantiomeren einer D,L-α-Hydroxycarbonsäure der Formel I oder eines Salzes davon durch asymmetrische Dehydrierung selektiv in die entsprechende α-Ketocarbonsäure oder ein Salz davon überzuführen, während das andere Enantiomere in Form einer optisch aktiven α-Hydroxycarbonsäure oder eines Salzes davon übrig bleibt. Der Mikroorganismus kann in Form der Gärbrühe oder in Form eines Extraktes davon verwendet werden.

Bevorzugte im erfindungsgemässen Verfahren verwendbare Stämme sind die folgenden, aus Bodenproben von verschiedenen Gegenden von Japan isolierten Bakterien und Actinomyceten, sowie mit diesen verwandte Stämme und Varianten davon. In der nachstehenden Tabelle sind die taxonomischen Merkmale solcher Bakterien sowie der Fundort angegeben. Alle in dieser Tabelle erwähnten Bakterien sind im Fermentation Research Institute, Agency of Industrial Science and Technology, Chiba, Japan unter den angegebenen FERM-P-Nummern deponiert worden. Entsprechende Kulturen sind ebenfalls in USA deponiert: die NRRL-Nummern beziehen sich auf in United States Department für Landwirtschaft, Northern Utilization Research and Development Division, Peoria, Illinois, deponierte Kulturen, die ATCC-Nummern auf Kulturen, welche in American Type Culture Collection, Rockville, Maryland, deponiert wurden.

A–1) Morphologische Eigenschaften und Kultureigenschaften von grampositiven, Sporen bildenden Stäbchen

| Eigenschaften \ Stämme | NRS–85KH20B (FERM–P No. 3164) (NRRL B–11084) | NRS–112KH25B (FERM–P No. 3165) (NRRL 11085) | NRS–137KH20B (FERM–P No. 3169) (ATCC 31301) |
|---|---|---|---|
| a) Morphologie | | | |
| 1 Form | Stäbchen | Stäbchen | Stäbchen |
| 2 Grösse ($\mu$) | 1,4–1,8 × 2,0–4,0 | 0,8–1,2 × 3,0–7,0 | 0,5–1,0 × 1,5–4,0 |
| 3 Pleomorphismus | – | – | – |
| 4 Motilität | (–) | + | + |
| 5 Sporen | + | + | + |
| (I) Grösse ($\mu$) | 0,8–1,0 × 1,5–2,0 | 0,8–1,0 × 1,5–2,0 | 0,4–0,8 × 0,8–1,2 |
| (II) Form | elliptisch | elliptisch | elliptisch |
| (III) Ausdehnung des Sporangiums | – | – | – |
| (VI) Position | Zentrum | Zentrum | Zentrum |
| 6 Gram-Färbung | + | + | + |
| 7 Säurefeste Färbung | – | – | – |
| b) Kultureigenschaften | | | |
| 1 Agar-Nährplatte | | | |
| Form | konvex, ganzrandig glatt | flach, wellig rauh | flach, schizoid rauh |
| Opazität | lichtdurchlässig | undurchsichtig | lichtdurchlässig |
| Farbe der Kolonie | hellgelb bis hellbraun | crème | hellgelb bis crème |
| Lösliches Pigment | bräunlich | keines | keines |
| 2 Schrägagarkultur | | | |
| Wachstum | reichlich | reichlich | mässig |
| Form | filiform | ausbreitend | stachelig |
| Glanz | matt | matt | matt |
| Farbe der Kolonie | crème bis hellbraun | weiss-crème | hellbraun bis orange |
| Lösliches Pigment | braun | keines | keines |
| 3 Bouillonflüssigkeit | | | |
| Wachstum | mässig | mässig | reichlich |
| Oberflächen-Wachstum | reichlich | keines | gering |
| Niederschlag | gering | reichlich | mässig |
| Pigmentation | keine | keine | keine |
| 4 Gelatine-Stichkultur | | | |
| Wachstum | reichlich | sehr reichlich | reichlich |
| Verflüssigung | + | + | + |
| 5 Lackmusmilch | | | |
| Lackmus | – | – | alkalisch |
| Peptonisierung | + | + | – |
| Koagulation | – | – | – |

A–2) Physiologische Eigenschaften von grampositiven, Sporen bildenden Stäbchen

| Eigenschaften / Stämme | NRS–85KH20B (FERM–P No. 3164) (NRRL B–11084) | NRS–112KH25B (FERM–P No. 3165) (NRRL 11085) | NRS137KH20B (FERM–P No. 3169) (ATCC 31301) |
|---|---|---|---|
| c) Physiologische Eigenschaften | | | |
| 1 Nitratreduktion | – | + | + |
| 2 Nitratrespiration | – | – | – |
| 3 MR–Test | – | + | – |
| 4 VP–Test | – | – | + |
| 5 Indolproduktion | – | – | – |
| 6 $H_2S$–Produktion | – | – | – |
| 7 Stärkehydrolyse | + | + | – |
| 8 Citratverwertung | | | |
| a. Koser-Medium | + | + | + |
| b. Christensen-Medium | + | – | + |
| 9 Verwertung von | | | |
| $NaNO_3$ | + | + | + |
| $(NH_4)_2SO_4$ | + | + | + |
| Harnstoff | + | + | + |
| Na-Glutamat | + | + | + |
| 10 Pigmentbildung | + (unlöslich) | – | – |
| 11 Urease | – | – | + |
| 12 Oxidase | + | + | + |
| 13 Katalase | + | + | + |
| 14 Wachstumbereich | | | |
| pH | 5,0–9,5 | 5,0–10,0 | 5,0–10,0 |
| Temperatur | 20°C–45°C | 20°C–37°C | 10°C–40°C |
| 15 Aerobes Wachstum | + | + | + |
| Anaerobes Wachstum | + | + | + |
| 16 O–F–Test | Fermentation | Fermentation | Fermentation |
| 17 Säure und Gas | Säure Gas | Säure Gas | Säure Gas |
| 1) L-Arabinose | + – | – – | – – |
| 2) D-Xylose | + – | – – | – – |
| 3) D-Glucose | + – | + – | – – |
| 4) D-Mannose | – – | – – | – – |
| 5) D-Fructose | + – | + – | – – |
| 6) D-Galactose | + – | – – | – – |
| 7) Maltose | + – | + – | – – |
| 8) Saccharose | + – | + – | – – |
| 9) Lactose | + – | – – | – – |
| 10) Trehalose | + – | + – | – – |
| 11) D-Sorbit | – – | – – | – – |
| 12) D-Mannit | + – | – – | – – |
| 13) Inosit | (+) – | + – | (–) – |
| 14) Glycerin | + – | + – | (–) – |
| 15) Stärke | + – | + – | – – |
| 18 Casein-Hydrolyse | + | + | + |
| 19 Tyrosin-Hydrolyse | / | / | + |
| 20 Phenylalanin-deaminase | / | / | + |
| Herkunft der Bodenprobe | Nerima-ku, Tokyo, Japan | Tobata-ku, Kita-Kyushu-shi, Japan | Kiyosato, Nagano Pref., Japan |

B–1) Morphologische Eigenschaften und Kultureigenschaften von pleomorphen Bakterion

| Eigenschaften / Stämme | NRS–112KH31B (FERM–P No. 3166) (NRRL B–11086) | NRS–129KH5B2 (FERM–P No. 3167) (ATCC 31300) | NRS–130–KH–20B (FERM–P No. 3657) (NRRL B–11088) | NRS–135KH9B (FERM–P No. 3168) (NRRL B–11087) |
|---|---|---|---|---|
| a) Morphologie | | | | |
| 1 Pleomorphismus | + | + | + | + |
| 2 Form | Stäbchen werden kokkoide Zellen | Stäbchen werden kokkoide Zellen | Stäbchen werden kokkoide Zellen | Stäbchen werden kokkoide Zellen |
| 3 Grösse (μ) 24 Std. | 0,5–1,0 × 3,0–5,0 | 0,5–1,0 × 3,0–5,0 | 0,5–1,0 × 3,0–5,0 | 1,2–1,5 × 2,5–6,0 |
| 48 Std. | 0,5–0,8 × 1,0–1,5 | 0,5–0,8 × 1,0–1,5 | 0,5–0,8 × 1,0–1,5 | 0,5–1,0 × 1,0–1,5 |
| 4 Motilität | – | – | – | – |
| 5 Gram-Färbung | + | + | + | + |
| 6 Säurefeste Färbung | – | – | – | – |
| 7 Sporen | – | – | – | – |
| b) Eigenschaften der Kultur | | | | |
| 1 Agar-Nährplatte | | | | |
| Form | konvex, ganzrandig glatt | konvex, ganzrandig glatt | konvex, ganzrandig glatt | konvex, ganzrandig glatt |
| Opazität | lichtdurchlässig | lichtdurchlässig | lichtdurchlässig | undurchsichtig |
| Farbe der Kolonie | crème | crème | crème-weiss | crème |
| Lösliches Pigment | keines | keines | keines | keines |
| 2 Schrägagarkultur | | | | |
| Wachstum | reichlich | reichlich | reichlich | reichlich |
| Form | Filiform | Filiform | Filiform | Filiform |
| Glanz | glänzend | glänzend | glänzend | glänzend |
| Farbe der Kolonie | crème-weiss | crème-weiss | crème-weiss | crème-weiss |
| Lösliches Pigment | keines | keines | keines | keines |
| 3 Bouillonflüssigkeit | | | | |
| Wachstum | mässig | mässig | mässig | reichlich |
| Oberflächen-Wachstum | keines | keines | keines | reichlich |
| Niederschlag | gering | gering | gering | mässig |
| Pigmentation | keine | keine | keine | keine |
| 4 Gelatine-Stichkultur | | | | |
| Wachstum | mässig bis reichlich | mässig bis reichlich | mässig bis reichlich | mässig |
| Verflüssigung | + | + | + | – |
| 5 Lackmusmilch | | | | |
| Lackmus | alkalisch | alkalisch | alkalisch | alkalisch |
| Peptonisierung | – | – | – | – |

B–2) Physiologische Eigenschaften von pleomorphen Bakterien

| Eigenschaften \ Stämme | NRS–112KH31B (FERM–P No. 3166) (NRRL B–11086) | NRS–129KH5B2 (FERM–P No. 3167) (ATCC 31300) | NRS–130KH20B (FERM–P No. 3657) (NRRL B–11088) | NRS–135KH9B (FERM–P No. 3168) (NRRL B–11087) |
|---|---|---|---|---|
| c) Physiologische Eigenschaften | | | | |
| 1 Nitratreduktion | – | – | – | + |
| 2 Nitratrespiration | – | – | – | – |
| 3 MR-Test | – | – | – | – |
| 4 VP-Test | – | – | – | – |
| 5 Indolproduktion | – | – | – | – |
| 6 $H_2S$-Produktion | – | – | – | – |
| 7 Stärkehydrolyse | – | – | – | – |
| 8 Citratverwertung | | | | |
| a. Koser-Medium | + | + | + | + |
| b. Christensen-Medium | + | + | + | + |
| 9 Verwertung von | | | | |
| $NaNO_3$ | + | + | + | + |
| $(NH_4)_2SO_4$ | + | + | + | + |
| Harnstoff | + | + | + | + |
| Na-Glutamat | + | + | + | + |
| 10 Pigmentbildung | – | – | – | – |
| 11 Urease | – | – | (–) | + |
| 12 Oxidase | – | – | – | – |
| 13 Katalase | + | + | + | + |
| 14 Wachstumbereich | | | | |
| pH | 5,0–9,5 | 5,0–9,0 | 5,0–10,0 | 5,0–10,0 |
| Temperatur | 15°C–37°C | 10°C–37°C | 10°C–37°C | 5°C–30°C |
| 15 Aerobes Wachstum | + | + | + | + |
| Anaerobes Wachstum | + | + | + | + |
| 16 O–F-Test | (Fermentation) | (Fermentation) | (Fermentation) | (Fermentation) |
| 17 Säure und Gas | Säure / Gas | Säure / Gas | Säure / Gas | Säure / Gas |
| 1) L-Arabinose | – / – | – / – | – / – | – / – |
| 2) D-Xylose | + / – | (+) / – | (+) / – | (+) / – |
| 3) D-Glucose | + / – | + / – | (+) / – | + / – |
| 4) D-Mannose | – / – | – / – | (+) / – | – / – |
| 5) D-Fructose | + / + | + / – | (+) / – | + / – |
| 6) D-Galactose | + / – | (+) / – | – / – | – / – |
| 7) Maltose | – / – | – / – | (+) / – | – / – |
| 8) Saccharose | – / – | – / – | (+) / – | + / – |

B–2) Fortsetzung

| Eigenschaften / Stämme | NRS–112KH31B (FERM–P No. 3166) (NRRL B–11086) | NRS–129KH5B2 (FERM–P No. 3167) (ATCC 31300) | NRS–130KH20B (FERM–P No. 3657) (NRRL B–11088) | NRS–135KH9B (FERM–P No. 3168) (NRRL B–11087) |
|---|---|---|---|---|
| 9) Lactose | – – | – – | – – | – – |
| 10) Trehalose | + – | (+) – | – – | + – |
| 11) D-Sorbit | + – | + – | – – | + – |
| 12) D-Mannit | – – | – – | – – | + – |
| 13) Inosit | – – | (+) – | (+) – | (+) – |
| 14) Glycerin | – – | + – | (+) – | + – |
| 15) Stärke | – – | – – | – – | – – |
| 18 Caseinhydrolyse | – | – | + | – |
| 19 DN-ase | – | – | + | – |
| 20 Wachstum in 5% NaCl | + | + | + | + |
| 21 Wachstum in 10% NaCl | – | – | – | – |
| Herkunft der Bodenprobe | Tobata-ku, Kita-kyushu, Japan | Shinano-Ohmachi Nagano-Pref., Japan | Fuji-shi, Shizu-oka-Pref., Japan | Shosenkyo, Yama-nashi-Pref., Japan |

C–1) Morphologische Eigenschaften und Eigenschaften der Kultur von gramnegativen Stäbchen

| Stämme / Eigenschaften | NRS–137CzH5B (FERM–P No. 3658) (NRRL 11089) | NRS–146KH6B (FERM–P No. 3172) (ATCC 31303) | NRS–140KH27B (FERM–P No. 3659) (NRRL 11090) | NRS–139KH6B (FERM–P No. 3170) (ATCC 31302) |
|---|---|---|---|---|
| a) Morphologie | | | | |
| 1 Form | Stäbchen | Stäbchen | Stäbchen | Stäbchen |
| 2 Grösse ($\mu$) | 0,4–0,6 × 1,5–2,5 | 0,5–0,8 × 1,5–3,0 | 0,5–0,8 × 2,0–3,5 | 0,4–0,7 × 1,0–1,5 |
| 3 Pleomorphismus | – | – | – | – |
| 4 Motilität | + | + | + | + |
| 5 Sporen | – | – | – | – |
| 6 Gram-Färbung | – | – | – | – |
| 7 Säurefeste Färbung | – | – | – | – |
| b) Eigenschaften der Kultur | | | | |
| 1 Agar-Nährplatte | | | | |
| Form | konvex, ganzrandig glatt | gebuckelt, ganzrandig runzlig | konvex, ganzrandig runzlig | konvex, ganzrandig glatt |
| | | | | |
| Opazität | lichdurchlässig | lichtdurchlässig | lichtdurchlässig | undurchsichtig |
| Farbe der Kolonie | hellgelb bis orange | hellgelb bis orange | hellgelb | crème-weiss |
| Lösliches Pigment | keines | keines | keines | keines |
| 2 Schrägagarkultur | | | | |
| Wachstum | reichlich | reichlich | reichlich | mässig |
| Form | Filiform | Filiform | Filiform | Filiform |
| Glanz | glänzend | glänzend | glänzend | glänzend |
| Farbe der Kolonie | hellbraun bis orange | hellbraun bis orange | hellbraun | crème-weiss |
| Lösliches Pigment | keines | keines | keines | keines |
| 3 Bouillonflüssigkeit | | | | |
| Wachstum | reichlich | reichlich | reichlich | mässig |
| Oberflächenwachstum | gering | reichlich | reichlich | reichlich |
| Niederschlag | mässig | gering | mässig | gering |
| Pigmentation | leicht gelblich | leicht gelblich | keine | keine |
| 4 Gelatine-Stichkultur | | | | |
| Wachstum | mässig bis reichlich | mässig | mässig | mässig |
| Verflüssigung | + | – | – | – |
| 5 Lackmusmilch | | | | |
| Lackmus | alkalisch | alkalisch | alkalisch | alkalisch |
| Peptonisation | + | – | – | – |
| Koagulation | – | – | – | – |

C–2) Physiologische Eigenschaften von gramnegativen Stäbchen

| Eigenschaften / Stämme | NRS–137CzH5B (FERM–P No. 3658) (NRRL 11089) | NRS–146KH6B (FERM–P No. 3172) (ATCC 31303) | NRS–140KH27B (FERM–P No. 3659) (NRRL 11090) | NRS–139KH6B (FERM–P No. 3170) (ATCC 31302) |
|---|---|---|---|---|
| c) Physiologische Eigenschaften | | | | |
| 1 Nitratreduktion | – | + | – | – |
| 2 Nitratrespiration | – | – | – | – |
| 3 MR-Test | – | – | – | |
| 4 VP-Test | – | – | – | – |
| 5 Indolproduktion | – | – | – | – |
| 6 $H_2S$-Produktion | – | – | – | – |
| 7 Stärkehydrolyse | – | – | – | – |
| 8 Citratverwertung | | | | |
| a. Koser-Medium | + | + | + | + |
| b. Christensen-Medium | + | + | + | + |
| 9 Verwertung von | | | | |
| $NaNO_3$ | + | + | + | + |
| $(NH_4)_2SO_4$ | + | + | + | + |
| Harnstoff | + | + | + | + |
| Na-Glutamat | + | + | + | + |
| 10 Pigmentbildung | fluoreszierend löslich | fluoreszierend löslich | fluoreszierend löslich | keine |
| 11 Urease | – | – | – | + |
| 12 Oxidase | + | + | + | + |
| 13 Katalase | + | + | + | + |
| 14 Wachstumbereich | | | | |
| pH | 5,0–10,0 | 5,0–10,0 | 5,0–9,0 | 5,0–9,5 |
| Temperatur | 5°C–30°C | 5°C–37°C | 5°C–37°C | 20°C–30°C |
| 15 Aerobes Wachstum | + | + | + | + |
| Anaerobes Wachstum | + | + | + | + |
| 16 O–F-Test | Oxydation | Oxydation | Oxydation | Oxydation |

C–2) Fortsetzung

| Eigenschaften / Stämme | NRS–137CzH5B (FERM–P No. 3658) (NRRL 11089) | | NRS–146KH6B (FERM–P No. 3172) (ATCC 31303) | | NRS–140KH27B (FERM–P No. 3659) (NRRL 11090) | | NRS–139KH6B (FERM–P No. 3170) (ATCC 31302) | |
|---|---|---|---|---|---|---|---|---|
| 17 Säure und Gas | Säure | Gas | Säure | Gas | Säure | Gas | Säure | Gas |
| 1) L-Arabinose | + | – | + | – | + | – | + | – |
| 2) D-Xylose | + | – | + | – | + | – | + | – |
| 3) D-Glucose | + | – | + | – | + | – | + | – |
| 4) D-Mannose | + | – | + | – | + | – | + | – |
| 5) D-Fructose | (–) | – | + | – | + | – | + | – |
| 6) D-Galactose | + | – | + | – | + | – | + | – |
| 7) Maltose | – | – | – | – | – | – | + | – |
| 8) Saccharose | – | – | – | – | (–) | – | + | – |
| 9) Lactose | (–) | – | – | – | – | – | + | – |
| 10) Trehalose | – | – | – | – | (–) | – | + | – |
| 11) D-Sorbit | (+) | – | – | – | (–) | – | + | – |
| 12) D-Mannit | (+) | – | – | – | (–) | – | + | – |
| 13) Inosit | (+) | – | – | – | (–) | – | + | – |
| 14) Glycerin | (+) | – | + | – | + | – | + | – |
| 15) Stärke | – | – | – | – | – | – | – | – |
| 18 Caseinhydrolyse | + | | – | | – | | – | |
| 19 Argininhydrolyse | + | | + | | + | | – | |
| Herkunft der Bodenprobe | Kiyosato, Nagano Pref., Japan | | Sarugakyo, Gumma Pref., Japan | | Shmada-shi Shizuoka Pref., Japan | | Kiyosato, Nagano Pref., Japan | |

Aus diesen Resultaten geht hervor, dass man diese Stämme anhand von Bergey's Manual of Determinative Bacteriology (8th Ed., 1974; 7th Ed., 1957); Riichi Sakazaki, "Identification of Medical Bacteria", Kindai Shuppan, 1971; und Kazuhiko Yamada and Kazuo Komagata., J. Gen. Appl. Microbiol., *18*, 417, 1972 wie folgt identifizieren kann:

1) Stamm FERM-P No. 3164 (NRRL B—11084):

Eine aus Glucose anaerob säurebildende Variante von Bacillu megaterium.

2) Stamm FERM-P No. 3165 (NRRL 11085):

Bacillus species, mit gewisser Aehnlichkeit mit B. circulans und B. firmus. Der Stamm unterscheidet sich jedoch vom ersteren Stamm durch die Morphologie der Sporen und vom letzteren Stamm durch das anaerobe Wachstum.

3) Stamm FERM-P No. 3169 (ATCC 31301):

Als Bacillus freudenreichii identifiziert.

4) Stämme FERM-P No. 3166 (NRRL B-11086), FERM-P No. 3167 (ATCC 31300), FERM-P No. 3657 (NRRL B-11088) und FERM-P No. 3168 (NRRL 11087):

Diese vier Stämme sind aerob bis faultativ anaerob, grampositiv, nichtbeweglich, nicht-sporenbildend und nicht-säurefest, gerade bis leicht gekrümmte Stäbchen in der ersten Wachstumsphase, und kugelförmig bis eiförmig nach mehr als 1 Tag Wäshstum. Diese Eigenschaften zeigen, dass alle diese Stämme der sogenannten Coryneform-Gruppe angehören. Bekanntlich (siehe z.B. Yamada, Komagata, J. Gen. Appl. Microbiol., *18*, 417, 1972) ist es schwierig, für diese Gruppe die Familie und Gattung anzugeben. Jedoch ist die folgende Indentifizierung anhand von Bergey's Manual möglich. Die verschiedenen Gattungen der Coryneform-Gruppe unterscheiden sich alle von der Gattung Arthrobacter durch die Säureproduktion aus Glucose, Fructose und anderen Zuckern, von der Gattung Kuruthia durch die fehlende Motilität, von der Gattung Microbacterium dadurch, dass alle diese Stämme beim Erhitzen in abgerahmter Milch auf 72°C während 15 Minuten nicht überleben, und von der Gattung Cellulomonas durch die Unfähigkeit, Cellulose zu verwerten.

Stamm Ferm-P No. 3168 (NRRL B-11087):

Aus den in der Tabelle angegebenen Eigenschaften dieses Stammes geht hervor, dass er mit Corynebacterium callunae, Brevibacterium lactofermentum, B. vitaruman und C. hydrocarboclastus sehr nah verwandt ist. Die drei letzten Stämme unterscheiden sich jedoch vom Stamm No. 3168 durch ihre pleomorphen Eigenschaften und durch Granulabildung. Trotz des Unterschiedes in der Verwetung von Zuckern und in der Säureproduktion ist der Stamm FERM-P No. 3168 (NRRL B-11087) am nächsten mit C. hydrocarboclastus verwandt und wurde daher als eine Variante von C. hydrocarboclastus identifiziert.

Stamm FERM-P No. 3657 (NRRL B-11088):

Die Eigenschaften dieses Stammes, nämlich fehlende Bildung von Granula, Säureproduktion, hydrolytische Aktivitäten gegen Gelatine und Casein, positive DN-ase, negative Urease und Fehlen von Wachstum in 10% NaCl lassen darauf schliessen, dass der Stamm No. 3657 mit Brevibacterium albidum sehr nah verwandt ist. Trotz einiger Unterschiede in der Säureproduktion aus D-Mannose, Saccharose, Inosit und Glycerin wurde der Stamm FERM-P No. 3657 (NRRL B-11088) auf Grund der Analogie zwischen allen anderen Eigenschaften als B. albidum identifiziert.

Stämme FERM-P No. 3166 (NRRL B-11086) und FERM-P No. 3167 (ATCC 31300):

Diese beiden Stämme sind sich sehr ähnlich und nicht voneinander unterscheidbar. Unter den in Bergey's Manual (7. und 8. Ausgabe) und Komagata et al. (J. Gen Appl. Microbiol., *18*, 399, 1972) beschriebenen Arten wurde Brevibacterium tegmenticola am nächsten verwandt gefunden. Da sich jedoch diese Stämme von B. tegmenticola durch die Grösse der Zelle, die Reaktionen in Lackmusmilch und die Säureproduktion aus Maltose und Saccharose unterscheiden, kann hier nicht von Identität gesprochen werden. Es scheint daher zweckmässig, diese Stämme als neue Arten, die eine gewisse Aehnlichkeit mit B. tegmenticola haben, anzusehen.

5) Stamm FERM-P No. 3658 (NRRL 11089):

Identifiziert als Pseudomonas fluorescens.

6) Stämme FERM-P No. 3172 (ATCC 31303) und FERM-P No. 3659 (NRRL 11090):

Identifiziert als Pseudomonas putida. Es wurden einige Unterschiede zwischen den Eigenschaften der beiden Stämme, wie z.B. der Nitratreduktion, festgestellt. Da man feststellen konnte, dass die Nitratreduktion bei 16—85% der Stämme dieser Art positiv war, kann der Stamm FERM-P No. 3659 (NRRL 11090) als eine Nitrat nicht-reduzierende Variante von Ps. putida angesehen werden.

7) Stamm FERM-P No. 3170 (ATCC 31302):

Pseudomonas species. Unter verschiedenen Pseudomonas-Stämmen ist der Stamm FERM-P No. 3170 (ATCC 31302) am nächsten mit Ps. cepacia verwandt. Auf Grund der Unterschiede in der Gelatineverflüssigung, der Caseinhydrolyse, des Wachstums bei 42°C und der Nitratreduktion kann der Stamm nicht als identisch mit Ps. cepacia angesehen werden.

Die isolierten Actinomyceten haben folgende Wachtumseigenschaften:

1) Stamm FERM-P No. 3160 (NRS-79KH-1A, NRRL 11083):

Dieser aus einer Bodenprobe von Uwajima-shi, Ehime Pref., Japan, isolierte Stamm entwickelt sich gut auf verschiedenen ISP-Agarmedien für Actionomyceten und bildet ein gutentwickeltes Luftmycel mit sporenbildenden Hyphen. Die Sporen, die in Form von Ketten von etwa 50 Sporen pro Kette

vorkommen, sind zylindrisch, haben eine stachlige Oberfläche und eine Grösse von 0,4~0,8 × 0,8~1,4 μ. Die Bildung von irgendeinem anderen spezifischen Organ wurde nicht beobachtet.

Auf den meisten getesteten Agarmedien ist das Wachstum (vegetatives Mycel) leicht bräunlichgrau bis hellbraun und das Luftmycel ist leicht grau bis grau. Das lösliche Pigment ist bräunlich bis rötlich. Die Bildung von Melanin-artigem Pigment wurde nicht beobachtet.

2) FERM-P No. 3600 (NRS-125KH-27A, NRRL 11091):

Dieser aus einer Bodenprobe von Suwa-shi, Nagano Pref., Japan isolierte Stamm entwickelt sich auch gut auf verschiedenen ISP-Agarmedien. Aus einem gut entwickelten vegetativen Mycel bildet sich ein relativ langes, gerades oder gekrümmtes Luftmycel. Weder Wirbel- noch Spiralbildungen werden beobachtet. Die Sporen, in Ketten von mehr als 50 Sporen pro Kette, sind zylindrisch, haben eine glatte Oberfläche und eine Grösse von 0,5—0,7 × 0,8—1,2 μ.

Das Wachstum ist schwach gelblich-braun bis schwach gelb und das Luftmycel ist weiss. Es bildet sich ein gelbliches lösliches Pigment. Es wurde kein Melanin-artiges Pigment beobachtet.

Aus den obigen Eigenschaften geht klar hervor, dass die Stämme FERM-P No. 3160 (NRRL 11083) und FERM-P No. 3660 (NRRL 11091) typische Streptomyces Species sind.

Die erfindungsgemässe asymmetrische Dehydrierung kann beispielsweise dadurch durchgeführt werden, dass man die Kulturbrühe oder kultivierte Zellen der Mikroorganismen mit einer DL-α-Hydroxycarbonsäure oder einem Salz hiervon zusammenbringt. Die Kulturbrühe kann durch Beimpfen eines zweckmässigen Mediums mit dem Mikroorganismus hergestellt werden. Das Kulturmedium kann beispielsweise Fleichextrakt, Hefeextrakt, Pepton, Maisquellwasser, weitere üblicherweise versendete Natursubstanzen oder Gemische davon, sowie Saccharide oder andere Kohlenstoffquellen, oder organische oder anorganische, Stickstoff enthaltende Verbindungen, wie Aminosäuren oder Salpetersäure, enthalten. Nötigenfalls kann das pH des Kulturmediums durch Zugabe von geeigneten Salzen, wie Natriumphosphat oder Natriumchlorid oder eines anderen Metallsalzes auf 7 gestellt werden. Es können Submerskulturen, Schüttelkulturen oder stationäre Kulturen verwendet werden. Die Kultivierung wird jedoch vorzugsweise unter aeroben Bedingungen durchgeführt. Die Kultivierungstemperatur liegt im allgemeinen im Bereich von 20—40°C, vorzugsweise im Bereich von 25—35°C. Die Kultivierung dauert zweckmässig 20—80 Stunden. Unter den oben beschriebenen Kultivierungsbedingungen erreicht das Wachstum det Stämme die stationäre Phase. Eine DL-α-Hydroxycarabonsäure der allgemeinen Formel I oder ein Salz hiervon kann als Substrat der Kulturbrühe zugesetzt werden, in der das Wachstum des Stammes die stationäre Phase erreicht hat. Die Konzentration des Substrats ist zweckmässig 1—200 mg/ml, vorzugsweise 3—120 mg/ml. Die asymmetrische Dehydrierung kann unter den oben beschriebenen Bedingungen durch Fortsetzung der Submerskultur, der Schüttelkulturen oder der stationären Kultur durchgeführt werden. Die Reaktionszeit hängt unter anderem von der Spezies und dem Stamm des verwendeten Mikroorganismus, der Zusammensetzung des Mediums, der Natur und der Konzentration des Substrats ab. Im allgemeinen genügt jedoch eine Reaktionszeit von 70—360 Stunden. Das Ende der Reaktion kann durch Messung der Menge an Reaktionsprodukt mittels, gas-chromatographischen oder kolorimetrischen Methoden, wie weiter unten beschrieben, bestimmt werden.

Die asymmetrische Dehydrierung kann unter den oben beschriebenen Bedingungen auch durch Zusatz des Substrats zum Kulturmedium und darauffolgendes Beimpfen mit den Mikroorganismen vorgenommen werden.

Die asymmetrische Dehydrierung kann ferner durch Zusammenbringen behandelter Zellsubstanz mit DL-α-Hydroxycarbonsäuren oder Salzen hiervon durchgeführt werden. Der Ausdruck "behandelte Zellsubstanz" bezeichnet alle Materialien, die durch Behandlung der Mikroorganismen erhalten wurden und fähig sind, die enantiospezifische Dehydrogenaseaktivität beizubehalten oder zu erhöhen. Solche Materialien sind beispielsweise das Mycel oder Zellen, die aus der Kulturbrühe isoliert und gewaschen wurden, oder lyophilisierte Pulver davon; in an sich bekannter Weise aus den kultivierten Zellen oder aus dem Mycel erhaltener zellfreier Extrakt; oder gereinigte oder teilweise gereinigte Dehydrogenasepräparate, welche in an sich bekannter Weise durch Reinigung aus den besagten zellfreien Extrakten erhalten wurden. Falls eine solche behandelte Zellsubstanz verwendet wird, kann die asymmetrische Dehydrierung in einer wässrigen Lösung, beispielsweise einer Pufferlösung oder einem frischem Medium, durchgeführt werden. Das pH der Reaktionslösung liegt gewöhnlich zwischen 7 und 8,5, insbesondere bei etwa 8. Die Reaktionstemperatur liegt zweckmässig bei 20—60°C, insbesondere bei 25—50°C. Das Ende dieser Reaktion kann durch Messung der Menge und der optischen Reinheit der zurückbleibenden α-Hydroxycarbonsäure sowie der Menge der in der Kulturbrühe oder dem Reaktionsgemisch gebildeten Ketocarbonsäure mittels gaschromatographischer oder kolorimetrischer Methoden bestimmt werden.

Durch die erfindungsgemässe asymmetrische Dehydrierung wird eines der Enantiomeren der DL-α-Hydroxycarbonsäure der allgemeinen Formel I oder ein Salz hiervon selektiv in die entsprechende α-Ketocarbonsäure oder ein Salz hiervon übergeführt, während das andere Enantiomere in Form der optisch aktiven α-Hydroxycarbonsäure oder eines Salzes davon zurückbleibt. Ob die zurückbleibende optisch aktive Substanz in D-oder L-Form vorliegt, hängt von der Natur des verwendeten Mikroorganismus ab.

Wünscht man die D-Form, können folgende Mikroorganismen verwendet werden:

- Bacillus megaterium (FERM—P No. 3164, NRRL B—11084)
- Bacillus sp. (FERM—P No. 3165, NRRL 11085)
- Pseudomonas fluorescens (FERM—P No. 3658, NRRL 11089)
- Brevibacterium sp. verwandt mit B. tegmenticola (FERM—P No. 3166, NRRL B—11086 und FERM—P 3167, ATCC 31300)
- Brevibacterium albidum (FERM—P No. 3657, NRRL B—11088)
- Streptomyces sp. (FERM—P No. 3160, NRRL 11083)

Wünscht man die L-Form, können folgende Mikroorganismen verwendet werden:

- Bacillus freudenreichii (FERM—P No. 3169, ATCC 31301)
- Pseudomonas putida (FERM—P No. 3172, ATCC 31303 und FERM—P 3659, NRRL 11090)
- Pseudomonas sp. (FERM—P No. 3170, ATCC 31302)
- Corynebacterium hydrocarboclastus var. (FERM—P No. 3168, NRRL B—11087)
- Streptomyces sp. (FERM—P No. 3660, NRRL 11091)

Falls die Reaktionsprodukte in Form von Salzen erhalten werden, können letztere durch Zusatz einer Säure in die freien optisch aktiven α-Hydroxycarbonsäure und α-Ketocarbonsäuren übergeführt werden.

Die optisch aktiven α-Hydroxycarbonsäuren und die α-Ketocarbonsäuren können in an sich bekannter Weise, beispielsweise auf Grund von Löslichkeitsunterschieden, durch chromatographische Fraktionierung mittels eines Ionenaustauscherharzes oder Silicagel oder fraktionierte Destillation, aus der Reaktionslösung isoliert werden. Die Verbindungen können auch mit Hydrazinen in an sich bekannter Weise behandelt werden, wobei die α-Ketocarbonsäuren selektiv in die Hydrazone übergeführt werden, welche durch übliche Franktionierungsmethoden abgetrennt werden können.

Nach dem erfindungsgemässen Verfahren werden optisch reine D- oder L-α-Hydroxycarbonsäuren und α-Ketocarbonsäuren mit hoher Ausbeute in einfacher Weise erhalten.

In einer bevorzugten Ausführungsform des erfindungsgemässen Verfahrens wird die erhaltene α-Ketocarbonsäure in an sich bekannter Weise hydriert und das Hydrierungsprodukt als Ausgangsmaterial in die asymmetrische Dehydrierung zurückgeführt. Die Hydrierung kann in an sich bekannter Weise, vorzugsweise mittels eines Katalysators, wie Raney-Nickel, durchgeführt werden. Falls bei der katalytischen Hydrierung Wasser als Lösungsmittel verwendet wird, wird der Katalysator abfiltriert und das Filtrat konzentriert und das Konzentrat kann dann direkt für die asymmetrische Dehydrierung verwendet werden.

Die nach dem erfindungsgemässen Verfahren erhaltenen α-Hydroxycarbonsäuren können als Zwischenprodukte für die Herstellung von pharmazeutischen Produkten, beispielsweise von Penicillinen (vgl. die US Patentschrift 3839322 und die Reissue davon Nr. 29003 sowie die US Patentschriften 3956323 und 3957758), Aminosäuren und dergleichen, sowie als biochemische Reagenzien verwendet werden. Als wichtige Vertreter der besagten α-Hydroxycarbonsäuren können die D- und die L-Milchsäure genannt werden.

Die in den nachstehenden Beispielen verwendeten Medien sind wässrige Medien folgender Zusammensetzung:

| Medium A (pH 7,0) | |
|---|---|
| Glucose | 0,1% |
| Pepton | 1,0% |
| Fleischextrakt | 0,3% |
| Natriumchlorid | 0,5% |

| Medium B (pH 7,0) | |
|---|---|
| Glucose | 0,1% |
| Maisquellwasser | 2,0% |

Die in den Beispielen angegebene Menge an verbleibender α-Hydroxycarbonsäure, deren optische Reinheit und die Menge an erhaltener α-Ketocarbonsäure kann durch folgende Methoden bestimmt werden.

1) Quantitative Bestimmung der 2-Hydroxycarbonsäuren in den Fermentationsbrühen:

2,0 ml Fermentationsbrühefiltrat werden in einem 1,6 g Ammoniumsulfat enthaltendem 10 ml Zentrifugenglas aufgenommen, mit 10%iger Schwefelsäure angesäuert und dann mit 2,0 ml Aethylacetat extrahiert. 1,5 ml des Aethylacetatextraktes werden unter vermindertem Druck eingedampft. Dem Rückstand werden 2 ml Toluol zugesetzt und das Gemisch wird erneut zur Trockene eingedampft. Der Rückstand wird in 0,2 ml Pyridin gelöst und 0,1 ml Benzoylchlorid werden zugesetzt. Nach 20 Minuten bei Raumtemperatur wird das Gemisch im Eisbad abgekühlt, mit 1,0 ml eines 0,4%

Diäthylterephthalat (interner Standard, I.S.) enthaltenden 5:1-Gemisches von Toluol und n-Propanol behandelt und 20 Minuten bei Raumtemperatur belassen. Nach Zusatz von 2,0 ml Wasser wird das Gemsich geschüttelt und dann 5 Minuten unter 3000 Umdrehungen pro Minute zentrifugiert Die Toluolschicht wird über wasserfreiem Natriumsulfat getrocknet und ein Teil der Lösung (gewöhnlich 2 $\mu$l) wird unter den folgenden Bedingungen durch Gaschromatographie analysiert:

| | |
|---|---|
| Temperatur: | Kolonne 185°C, Injektor 225°C, Detektor 205°C |
| Säule: | Glasschlange<br>Länge 2 m<br>interner Durchmesser 3 mm |
| Packung der Säule: | 5% QF—1/chromosolve W, MMCS<br>80—900 mesh |
| Trägergas: | 30 ml/min Helium |
| Detektion: | — FID |

Die Retentionszeiten des I.S. und der Derivate der $\alpha$-Hydroxycarbonsäuren, z.B. der $\alpha$-Hydroxy-$\gamma$-methylvaleriansäure und der $\alpha$-Hydroxyhexansäure, sind unter den obigen Bedingungen 4,4, 6,6 bzw. 7,8 Minuten. Die den Peaks zugehörigen Flächen werden mittels der Eichkurve in Konzentrationswerte umgerechnet.

2) Bestimmung der optischen Reinheit der $\alpha$-Hydroxycarbonsäuren in Fermentationsbrühen:

7 ml des Fermentationsbrühefiltrats werden mit 1 ml 10%iger Schwefelsäure angesäuert und mit 5 ml Aethylacetat extrahiert. 4 ml Teilmengen des Aethylacetatextrakts werden unter vermindertem Druck zur Trockene eingedampft. Der Rückstand wird mit 2 ml 4% HCl enthaltendem n-Propanol behandelt. Nach 3 Stunden bei Raumtemperatur wird das Gemisch unter vermindertem Druck eingedampft und die letzten Spuren von n-Propanol werden mittels Toluol entfernt. Dem Rückstand werden 0,2 ml L-(—)-Methyloxycarbonylchloridlösung (hergestellt nach der Methode von J. W. Westley, J. Org. Chem. *33,* 3798, 1968) und 0,2 ml Pyridin zugesetzt. Nach 30 Minuten bei Raumtemperatur wird das Gemisch mit 2 ml kaltem Wasser behandelt. Dann werden 0,8 ml Toluol zugesetzt. Das Gemisch wird geschüttelt und zentrifugiert. Nach Trocknen mit Natriumsulfat wird ein Teil der Toluolphasen unter den gleichen Bedingungen wie für die quantitative Bestimmung der $\alpha$-Hydroxycarbonsäuren durch Gaschromatographie analysiert. Beispiele von Retentionszeiten für einige aus $\alpha$-Hydroxycarbonsäuren abgeleitete diastereomere Carbonate sind aus der nachstehenden Tabelle ersichtlich. Die optische Reinheit kann mittels folgender Gleichung ermittelt werden:

AL: Fläche des Peaks des L-Enantiomers
AD: (Fläche des Peaks des D-Enantiomers)/k
k: Verhältnis zwischen den Detektorangaben der Diastereomeren (LD/LL)

$$\text{Optische Reinheit} = \frac{|AL - AD|}{AL + AD} \times 100\ (\%)$$

| R in der Formel I | Retentionszeit des L-Enantiomeren | Retentionszeit des D-Enantiomeren | k |
|---|---|---|---|
| $CH_3(CH_2)_2$ | 8,5 (Min) | 9,2 (Min) | 0,96 |
| $(CH_3)_2CHCH_2$ | 8,8 | 9,6 | 1,00 |
| $CH_3(CH_2)_3$ | 10,8 | 11,8 | 1,16 |
| $CH_3(CH_2)_4$ | 14,0 | 15,3 | 1,14 |
| $CH_3(CH_2)_5$ | 18,4 | 20,2 | 1,17 |
| $CH_3(CH_2)_6$ | 24,5 | 26,7 | 1,15 |
| $CH_3(CH_2)_7$ | 32,4 | 35,3 | 1,05 |
| $CH_3(CH_2)_9$ | 57,8 | 63,0 | 1,00 |

3) Quantitative Bestimmung der $\alpha$-Ketocarbonsäuren

Es wird die kolorimetrische Methode von H. Katsuki et al., Anal. Biochem. *43,* 349 (1971) verwendet.

Beispiel 1

Vier mit Schikane versehene 500 ml Erlenmeyer-Kolben mit je 100 ml des Mediums A wurden im Autoklav behandelt und dann mit Bacillus sp. NRS—112KH25B (FERM—P No. 3165, NRRL 11085) aus der Schrägkultur beimpft. Die Inkubation wurde 26 Stunden bei 27°C unter Schütteln (180 Bewegungen pro Minute) durchgeführt. Danach wurden jedem Kolben 3,54 g Natrium-DL-2-hydroxy-4-methylvalerianat zugesetzt und die Fermentation wurde 84 Stunden bei 27°C unter Schütteln fotgeführt. Mittels der oben beschriebenen gaschromatographischen Methode wurde gefunden, dass das in der Kulturbrühe zurückbleibende Substrat 15,5 mg/ml freie D-Hydroxysäure (optische Reinheit: 100%) enthielt.

Die so erhaltene Gärbrühe (390 ml) wurde mit 50 ml 20%iger Schwefelsäure angesäuert und mit 300 ml Aethylacetat extrahiert. Die wässrigen Schichten wurden nochmals mit 100 ml Aethylacetat extrahiert. Die vereinigten Aethylacetatextrakte wurden über wasserfreiem Natriumsulfat getrocknet und unter vermindertem Druck eingeengt. Nach Kristallisation des Rückstandes aus Diäthyläther-Petroläther erhielt man 4,95 g D-2-Hydroxy-4-methylvaleriansäure, Schmelzpunkt 79,6°C, $[\alpha]_D^{25} = +26,3°$ (c=2, 1N NaOH), durch Massenspektrometrie mit hoher Auflösung ermittelte Bruttoformel: $C_6H_{12}O_3$.

Die Mutterlauge wurde unter vermindertem Druck eingeengt und der gelbliche braune, ölige Rückstand der fraktionierten Destillation unterworfen, wobei man 3,6 g 4-Methyl-2-oxo-valeriansäure in Form eines farblosen Oels erhielt, Siedepunkt 65—75°C (10 mmHg), durch Massenspektrometrie mit hoher Auflösung ermittelte Bruttoformel: $C_6H_{10}O_3$.

Beispiel 2

Ein mit Schikane versehener 500 ml Erlenmeyer-Kolben mit 90 ml des Mediums A wurde mit Bacillus freudenreichii NRS137KH20B (FERM—P No. 3169, ATCC 31301) von der Schrägkultur beimpft. Die Inkubation wurde unter den in Beispiel 1 beschriebenen Bedingungen 24 Stunden durchgeführt. Danach wurden 3,17 g Natrium-DL-2-hydroxy-4-methylvalerianat zugesetzt und die Gärung wurde 82 Stunden fortgesetezt. Das in der Fermentationsbrühe zurückbleibende Substrat enthielt 15,2 mg/ml freie L-Hydroxysäure, optische Reinheit 100%. Die so erhaltene Gärlösung wurde dann in zu Beispiel 1 analoger Weise behandelt und man erhielt 1,18 g kristalline L-2-Hydroxy-4-methylvaleriansäure, Schmelzpunkt 79,1°C, $[\alpha]_D^{25} = -26,8°$ (c=2, 1N NaOH) und 0,7 g 4-Methyl-2-oxovaleriansäure, Sidepunkt 65—75°C (10 mmHg).

Beispiel 3

Zehn mit Schikane versehene 500 ml Erlenmeyer-Kolben, ententhaltend je 100 ml des Mediums B, wurden mit Coryneform-Stamm NRS—130KH20B (FERM—P No. 3657, NRRL B—11088) von der Schrägkultur beimpft und 24 Stunden unter den gleichen Bedingun wie in Beispiel 1 bebrütet. Jedem Kolben wurden als Substrat 8,19 g Natrium-DL-2-hydroxy-4-methylvalerianat zugesetzt und die Gärung wurde 120 Stunden unter den gleichen Bedingungen fortgesetzt. Nach der oben beschriebenen Methode wurde gefunder dass 120 Stunden nach Zusatz des Substrats 35 mg/ml 4-Methyl-2-oxo-valeriansäure und 37 mg/ml reine D-Hydroxysäure vorlagen. Die Gärung wurde daher abgestellt und die Gärlösungen wurden kombiniert und zentrifugiert. Der Ueberstand wurde mit 38 ml 50%iger Schwefelsäure auf pH 1,5 angesäuert und dreimal mit 1 Liter Diäthyläther extrahiert. Die vereinigten Aetherextrakte wurden dreimal mit 10 ml gesättigter Natriumchloridlösung gewaschen und unter vermindertem Druck eingedampft. Dem Rückstand wurden 100 ml Toluol zugesetzt und das Gemisch wurde unter vermindertem Druck zwecks Entfernung von Wasser eingeengt. Der gelblich-braune, ölige Rückstand wurde in 400 ml Petroläther gelöst und die Lösung über Nacht im Kühlschrank gehalten. Das Kristallisat wurde durch Filtrieren gesammelt, mit 50 ml kaltem Petroläther gewaschen und im Vakuum getrocknet. Man erhielt 28,1 g D-2-Hydroxy-4-methyl-valeriansäure in Form von farblosen Kristallen, Schmelzpunkt 79,9°C, $[\alpha]_D^{26}=+26,67°$ (c=2, 1N NaOH).

Beispiel 4

Die 4-Methyl-2-oxovaleriansäure und eine kleine Menge D-2-Hydroxy-4-methylvaleriansäure enthaltende Mutterlauge und Waschwässer aus Beispiel 3 wurden vereinigt und in einen Scheidetrichter verbracht. Dann wurde unter Schütteln 1N NaOH zugesetzt bis das pH der wässrigen Schichten 7,0 erreichte (Zugabe von 299 ml 1N NaOH). Die wässrige Schicht wurde abgetrennt und unter vermindertem Druck auf etwa 150 ml eingeengt. Dem Rückstand wurden 100 ml destilliertes Wasser, enthaltend Raney-Nickel T4 (hergestellt aus 36 g einer 42% Nickel enthaltenden Nickel/Aluminiumlegierung nach der Methode von Nishimura, Bull. Chem. Soc. Japan, 32, 61—64, 1959), zugesetzt. Das Gemisch wurde bei Raumtemperatur unter normalem Druck bis zum Ende der Wasserstoffaufnahme hydriert. Der Katalysator wurde abfiltriert. Das Filtrat wurde mit 25 ml 50%iger Schwefelsäure angesäuert und dann veirmal mit 500 ml Aethyläther extrahiert. Die Aetherextrakte wurden vereinigt, mit 10 ml gesättigter Natriumchloridlösung gewaschen und unter vermindertem Druck eingeengt. Dem Rückstand wurden 100 ml Toluol zugesetzt und das Gemisch wurde nochmals unter vermindertem Druck zu einem leicht gelben Oel eingeengt. Zwecks Kristallisation wurden 160 ml Petroläther zugesetzt. Das Gemisch wurde über Nacht im Kühlschrank behalten, dann filtriert und die Kristalle wurden mit kaltem Petroläther gewaschen, wobei man 32,3 g 2-Hydroxy-4-methylvaleriansäure in Form von fablosen Kristallen erhielt Schmelzpunkt 73,9°C, $[\alpha]_D^{20}=+4.24°$ (c=2, 1N NaOH).

Beispiel 5

DL-2-Hydroxy-4-methylvaleriansäure wurde bis zu einer Konzentration von 3,0 mg/ml im Medium A gelöst und das pH durch Zugabe von 5N NaOH auf 7,0 gestellt. Drei 500 ml Erlenmeyer-Kolben, enthaltend je 100 ml des so hergestellten Mediums wurden im Autoklaven sterilisiert und dann mit einem der folgenden Stämme beimpft: Bacillus megaterium NRS—85KH20B (FERM—P No. 3164, NRRL B—11084), Pseudomonas fluorescens NRS—127CzH5B (FERM—P No. 3658, NRRL 11089) und Streptomyces sp. NRS79KH1A (FERM—P No. 3160, NRRL 11083). Die Kultivierung wurde unter Schütteln (180 Bewegungen/Minute) bei 27°C durchgeführt. Nach 72 und 144 Stunden wurde das Substrat, wie oben beschrieben, gaschromatographisch analysiert. Es wurden folgende Resultate ermittelt:

| Stämme | Menge des zurückbleibenden Substrats (optische Reinheit als D-Enantiomer) | | |
|---|---|---|---|
| | O Stunden (Beginn der Kultivierung) | 72 Stunden | 144 Stunden |
| FERM-P No. 3164, NRRL B-11084 | 3,0 mg/ml (0%) | 1,9 mg/ml (76%) | 1,8 mg/ml (92%) |
| FERM-P No. 3658, NRRL 11089 | 3,0 (0%) | 1,2 (100%) | 1,1 (100%) |
| FERM-P No. 3160, NRRL 11083 | 3,0 (0%) | 1,2 (100%) | 1,2 (100%) |

Beispiel 6

Nach der Methode von Beispiel 5 wurden folgende Stämme im Medium A, das 3,0 mg/ml DL-2-Hydroxy-4-methylvaleriansäure enthielt, kultiviert: Bacillus freudenreichii NRS—137KH20B (FERM—P No. 3169, ATCC 31301), Pseudomonas putida NRS—146KH6B (FERM—P No. 3172, ATCC 31303), Pseudomonas sp. NRS—139KH6B (FERM—P No. 3170, ATCC 31302), Coryne-Form NRS—135KH9B (FERM—P No. 3168, NRRL B—11087) und Streptomyces sp. NRS—125KH27A (FERM—P No. 3660, NRRL 11091). Die Gärlösung wurde unter Verwendung der oben angegebenen Methode nach 72 und 144 Stunden analysiert. Die Resultate sind in der folgenden Tabelle angegeben:

| Stamm | Zurückbleibende Menge des Substrats (optische Reinheit als L-Enantiomer) | | |
|---|---|---|---|
| | 0 Stunden (Beginn der Kultur) | 72 Stunden | 144 Stunden |
| FERM-P No. 3169, ATCC 31301 | 3,0 mg/ml (0%) | 1,6 g/ml (93%) | 1,4 mg/ml (100%) |
| FERM-P No. 3172, ATCC 31303 | 3,0 (0%) | 1,4 (98%) | 1,4 (100%) |
| FERM-P No. 3170, ATCC 31302 | 3,0 (0%) | 1,3 (93%) | 1,0 (100%) |
| FERM-P No. 3168, NRRL B-11087 | 3,0 (0%) | 1,6 (83%) | 0,6 (100%) |
| FERM-P No. 3660, NRRL 11091 | 3,0 (0%) | 1,3 (97%) | 1,2 (97%) |

Beispiel 7

Ein mit Schikane versehener, 100 ml Medium A enthaltender Erlenmeyer-Kolben wurde mit einem der Stämme FERM—P No. 3166, (NRRL B—11086), FERM—P No. 3657 (NRRL B—11088),

FERM—P No. 3658 (NRRL 11089), FERM—P No. 3167 (ATCC 31300), FERM—P No. 3659 (NRRL 11090) und FERM—P No. 3170 (ATCC 31302) ab einer 2—10 Tage alten Agar-Schrägkultur beimpft und 24—72 Stunden unter den gleichen Bedingungen wie in Beispiel 2 kultiviert. Die Kulturbrühe wurde 10 Minuten unter Kühlung zentrifugiert und die ausgeschiedenen Zellen wurden zweimal mit 100 ml 0,85% NaCl enthaltendem Phosphatpuffer (pH 7,0) gewaschen und dann in 100 ml dieses Puffers suspendiert. Die Zellsuspension wurde in 5-ml-Portionen mit DL-$\alpha$-Hydroxycarbonsäure versetzt, sodass die Konzentration 20 mg/ml betrug. Das Reaktionsgemisch wurde 20 Minuten bei 27°C inkubiert. Die Aktivität jedes Organismus für die verschiedenen Substrate wurde durch die oben beschriebene spektrophotometrische Analyse der entsprechenden entstandenen 2-Oxocarbonsäure ermittelt. Die Resultate sind in der nachstehenden Tabelle angegeben, worin die Reaktivität als relative Reaktivität gegenüber dem Substrat DL-2-Hydroxy-4-methylvaleriansäure (100) ausgedrückt ist. Die spezifische Aktivität jedes Stamms mit 2-Hydroxy-4-methylvaleriansäure ist: 5,8; 6,0; 0,95; 5,5; 3,5 und 0,54 nMol/min/mg Protein mit den Stämmen FERM—No. 3166, 3657, 3658, 3167, 3170 bzw. 3659.

Die Zellen wurden durch Beschallung (70 W, 15 Minuten) aufgebrochen und 10 Minuten unter Kühlung zentrifugiert. Der erhaltene Ueberstand (Rohextrakt), wurde wie oben beschrieben, mit verschiedenen Substraten in einem 0,02 M Phosphatpuffer (pH 7,0) oder einem 0,02 M Trishydrochloridpuffer (pH 8,0) inkubiert. Es wurden ähnliche relative Aktivitäten gefunden, obwohl die spezifische Aktivität kleiner war, z.B. 0,50 nMol/Min/mg Protein mit dem Stamm No. 3657.

| FERM–P No. / Hydroxy-säure = R | Relative Aktivität in jedem Stamm | | | | | |
|---|---|---|---|---|---|---|
| | 3166 | 3657 | 3658 | 3167 | 3170 | 3659 |
| | D | D | D | D | L | L |
| $CH_3-$ | – | – | 1310 | 159 | – | – |
| $CH_3CH_2-$ | 1,9 | – | 560 | 14 | 41 | – |
| $CH_3(CH_2)_2-$ | 32 | 32 | 130 | 42 | 150 | – |
| $(CH_3)_2CH-$ | 69 | 16 | 19 | 25 | – | – |
| $CH_3(CH_2)_3-$ | 72 | 68 | 194 | 59 | 39 | 400 |
| $(CH_3)_2CHCH_2-$ | 100 | 100 | 100 | 100 | 100 | 100 |
| $(CH_3)_3C-$ | – | – | – | – | – | 71 |
| $CH_3(CH_2)_4-$ | 120 | 133 | 200 | 80 | 30 | 1220 |
| $CH_3(CH_2)_5-$ | 68 | 79 | 160 | 49 | 13 | 693 |
| $CH_3(CH_2)_6-$ | 52 | 55 | 53 | 25 | 12 | 250 |
| $CH_3(CH_2)_7-$ | 39 | 13 | 23 | 6,5 | 6,8 | – |
| $CH_3(CH_2)_9-$ | 3,5 | 0,6 | 14 | 6,3 | 0,9 | – |
| $CH_3(CH_2)_{13}-$ | 3,4 | – | – | 14 | – | – |

Substrat: (DL) $R-CH(OH)-COOH$

Beispiel 8

Ein mit Schikane versehener, 100 ml Medium A enthaltender Erlenmeyer-Kolben wurde mit Brevibacterium albidum NRS—130KH20B (FERM—P No. 3657, NRRL B—11088) von der Schrägkultur beimpft und unter den gleichen Bedingungen wie in Beispiel 2 24 Stunden kultiviert. Die Kulturbrühe (90 ml) wurde zu 22 ml sterilisiertem 50%igem Glycerin gegeben, suspendiert und bei —196°C in flüssigem Stickstoff aufbewahrt.

Ein mit Schikane versehener, 100 ml des Mediums B enthaltender Erlenmeyer-Kolben wurde durch Zugabe von 2 ml der obigen, aufgetauten Zellsuspension beimpft und 24 Stunden unter identischen Bedingungen kultiviert. Dieser Kultur wurden 8,8 g Natrium-DL-2-hydroxy-4-methyl-

valerianat als Substrat zugesetzt und die Fermentation wurde fortgesetzt. 72 und 120 Stunden nach Zusatz des Substrats wurden die zurückbleibenden Mengen des Substrats in der Brühe, die optische Reinheit (ausgedrückt in % des D-Enantiomeren) und die Menge der erthaltenen 4-Methyl-2-oxo-valeriansäure durch die oben beschriebenen Methoden ermittelt. Die Resultate sind in der folgenden Tabelle angegeben:

| | Beim Zusatz des Substrats | 72 Stunden | 120 Stunden |
|---|---|---|---|
| 2-Hydroxy-4-methylvalerian-säure (mg/ml) | 75 | 41 | 39 |
| Optische Reinheit des D-Enantiomeren in % | 0 | 85 | 100 |
| 4-Methyl-2-oxovalereian-säure (mg/ml) | 0 | 32 | 33 |

Beispiel 9

Ein 1 Liter-Glasfermentor mit 300 ml des Mediums B wurde sterilisiert, dann mit 6 ml der wie in Beispiel 8 hergestellten Zellsuspension No. 3657 beimpft und 16 Stunden bei 27°C unter aeroben Bedingungen (Luftströmung 0,5 1/Min, 300 Umdrehungen/Minute) kultiviert. Als Substrat wurden 24,6 g Natrium-2-hydroxy-4-methylvalerianat zugesetzt und das pH der Kultur durch Zusatz von 2N HCl auf 8,5 eingestellt. Die Fermentation wurde bei pH 8,5 unter den gleichen Bedingungen fortgeführt (Luftströmung 0,3 1/Min, 300 Umdrehungen/Minute). Die Analyse des Substrats, 72 und 120 Stunden nach Substratzugabe, ist in der folgenden Tabelle angegeben:

| | Bei Zugabe des Substrats | 72 Stunden | 120 Stunden |
|---|---|---|---|
| 2-Hydroxy-4-methylvalerian-säure (mg/ml) | 70 | 39 | 35 |
| Optische Reinheit des D-Enantiomeren in % | 0 | 89 | 98 |
| 4-Methyl-2-oxovalerian-säure (mg/ml) | 0 | 36 | 36 |

Beispiel 10

Ein mit Schikane versehener 500 ml Erlenmeyer-Kolben mit 100 ml des Mediums B wurde mit 1 ml der gemäss Beispiel 8 hergestellten Zellsuspension von B. albidum NRS—130KH20B (FERM—P No. 3657, NRRL B—11088) beimpft und 24 Stunden bei 27°C unter Rühren und den in Beispiel 2 beschriebenen Bedingungen kultiviert. Dann wurden 1,48 g des Natriumsalzes der DL-$\alpha$-Hydroxy-3-pyridylessigsäure zugesetzt und die Fermentation wurde 120 Stunden fortgeführt. Die Kulturbrühe (pH 9,2) wurde zentrifugiert und durch eine Säule mit 71 ml Dowex-1x4 (Acetatform, 200—400 mesh) geführt. Der Anionenaustauscher wurde dann mit Wasser gewaschen und mit einem Gemisch von 2N Essigsäure/Methanol (1:1 v/v) eluiert. Das Eluat wurde durch Dünnschichtchromatographie (Platte: Silicagel, Lösungsmittel: Aethylacetat-Essigsäure-Wasser-Methanol, 10:2:1:2, v/v; Nachweis: UV-Strahlen) kontrolliert. Die UV-Absorptionsflecken (Rf 0,12) aufweisenden Fraktionen wurden vereinigt und unter vermindertem Druck eingeengt. Der Rückstand wurde in 20 ml Wasser gelöst und zu 793 mg Pulver lyophilisiert. Nach Kristallisation aus Methanol-Aethylacetat erhielt man 625 mg optisch aktive $\alpha$-Hydroxy-3-pyridylessigsäure in Form von farblosen Kristallen, Schmelzpunkt 140°C, $[\alpha]_D^{24}$=—119° (c=1, $H_2O$), Mol. Formel: $C_7H_7NO_3$.

Die Säule wurde dann mit einem Gemsich von 1N HCl und Aceton (1:1, v/v) eluiert. Das Eluat wurde nach dem gleichen Dünnschichtchromatographiesystem kontrolliert. Die UV-Absorptionsflecken (Rf 0,37) aufweisenden Fraktionen wurden vereinigt, mit Wasser verdünnt und dann durch eine Säule, enthaltend 30 ml Dowex-50 ($H^+$-Form), gegeben. Die Säule wurde mit Wasser gewaschen und mit 1%igem wässrigem Ammoniak eluiert. Das Eluat wurde unter vermindertem Druck eingeengt, lyophilisiert und das entstandene Pulver aus Methanol kristallisiert. Man erhielt 326 mg $\alpha$-Oxo-3-pyridylessig-säure in Form von farblosen Kristallen, Schmelzpunkt 177—179°C (Zers.), Mol. Formel: $C_7H_5NO_3$.

Beispiel 11

Ein mit Schikane versehener 500 ml Erlenmeyer-Kolben, enthaltend 100 ml des Mediums B, wurde mit 2 ml der nach Beispiel 8 hergestellten Zellsuspension von B. albidum NRS—130KH20B (FERM—No. 3657, NRRL B—11088) beimpft und 24 Stunden bei 27°C unter Schütteln (180 Bewegungen/Minute) kultiviert. Die Kulturbrühe wurde 10 Minuten zentrifugiert, die ausgeschiedenen Zellen wurden dreimal mit 100 ml 0,85% Natriumchlorid enthaltendem 0,02M Phosphatpuffer (pH 7,5) gewaschen und dann in 100 ml des gleichen Puffers suspendiert. 50 ml dieser Suspension wurden in einen 500 ml Erlenmeyer-Kolben umgeschüttet, mit 1,14 g Natrium DL-2-Hydroxyoctanoat versetzt und der Kolben 48 Stunden bei 27°C geschüttelt. Die Zellen wurden durch Zentrifugieren aus dem Reaktionsgemisch entfernt, der Ueberstand durch Zusatz von 50%iger Schwefelsäure auf pH 1,5 angesäuert und dreimal mit 50 ml Diäthyläther extrahiert. Die vereinigten Aetherextrakte wurden mit Wasser gewaschen und unter vermindertem Druck eingedampft. Der Rückstand wurde mit 26 ml 2,4-Dinitrophenylhydrazin-Reagens, hergestellt nach der Methode von C.D. Johnson, J. Am. Chem. Soc. *73,* 5888, (1951), versetzt und das Gemisch über Nacht bei Raumtemperatur stehen gelassen. Die entstandenen gelben Kristalle wurden gesammelt und aus Benzol umkristallisiert. Man erhielt 761 mg 2,4-Dinitrophenylhydrazon der 2-Oxooctansäure, Schmelzpunkt 137°C, Mol. Formel: $C_{14}H_{18}N_4O_6$.

Die Hydrazon-Mutterlauge wurde zweimal mit 50 ml Diäthyläther extrahiert. Die vereinigten Aetherextrakte wurden mit einer kleinen Menge Wasser gewaschen und das pH de wässrigen Schicht unter Schütteln und Zugabe von 1N NaOH auf 7,0 eingestellt. Die wässrige Schicht wurde zweimal mit 20 ml Diäthyläther gewaschen und unter vermindertem Druck eingeengt. Das Konzentrat wurde zu 237 mg Natrium D-2-Hydroxyoctanoat, $[\alpha]_D^{20}=+9,6°$ (c=2, $H_2O$) lyophilisiert.

Beispiel 12

Die Spaltung von verschiedenen DL-$\alpha$-Hydroxycarbonsäuren wird wie in Beispiel 11 mit den restlichen Zellen von B. albidum NRS—130KH20B (FERM—P No. 3657, NRRL B—11088) durchgeführt. Die Resultate sind in der folgenden Tabelle angegeben:

| Substrat | Zugesetzte Menge (mg) | Inkubierungszeit (Std.) | D-α-Hydroxycarbonsäure | | | α-Ketocarbonsäure | |
|---|---|---|---|---|---|---|---|
| | | | Ausbeute*1 (mg) | $[\alpha]_D^{20}$ *1 (c=2, $H_2O$) | Optische*2 Reinheit | Ausbeute*3 (mg) | Schmelzpunkt |
| 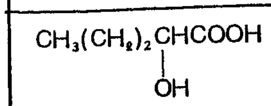 | 1510 | 72 | 474 | +10,1° | 90 | 405*4 | 166,5–167 |
| 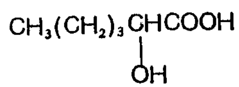 | 890 | 48 | 389 | +12.1° | 100 | 368*4 | 134 |
| 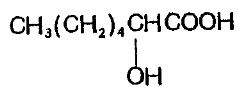 | 820 | 48 | 372 | +12,5° | 100 | 440*5 | 103–104 |
| 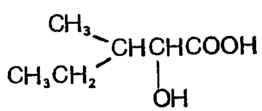 | 840 | 72 | 200 | +8,9° | 96 | 619*4 | 169–171 |

*1: als Natriumsalz

*2: gaschromatographisch ermittelt

*3: als 2,4-Dinitrophenylhydrazon

*4: umkristallisiert aus Benzol

*5: umkristallisiert aus Benzol-Hexan

Beispiel 13

Das gewaschene Zellpräparat, hergestellt aus 100 ml der nach Beispiel 11 hergestellten Kultur von B. albidum NRS—130KH20B (FERM—P No. 3657, NRRL B—11088) wurde nochmals mit destilliertem Wasser gewaschen und lyophilisiert, wobei man 478 mg Lyophilisat (trockenes Zellpulver) erhielt. Das trockene Zellpulver wurde in 100 ml 0,02 M Phosphatpuffer (pH 7,5) suspendiert. Das Gemisch wurde mit 2,34 g Natrium-DL-2-hydroxy-4-methylvalerianat versetzt und nach Einstellen des pH auf 7,5 bei 27°C geschüttelt (180 Bewegungen/Minute). Nach 48 und 72 Stunden Schütteln wurde das im Puffer zurückbleibende Substrat, die optische Reinheit und die Menge der erhaltenen 4-Methyl-2-oxovaleriansäure durch die oben beschriebene Methode ermittelt. Die Resultate sind in der folgenden Tabelle angegeben:

| | Vor dem Schütteln | 48 Stunden | 72 Stunden |
|---|---|---|---|
| 2-Hydroxy-4-methylvaleriansäure (mg/ml) | 20,0 | 11,6 | 10,9 |
| Optische Reinheit des D-Enantiomeren in % | 0 | 100 | 100 |
| 4-Methyl-2-oxovaleriansäure (mg/ml) | 0 | 9,6 | 9,9 |

## Patentansprüche

1. Verfahren zur Herstellung von optisch aktiven $\alpha$-Hydroxycarbonsäuren, dadurch gekennzeichnet, das man eine D,L-$\alpha$-Hydroxycarbonsäure der allgemeinen Formel

$$R—CH(OH)—COOH \quad I$$

worin R einen verzweigten oder geradkettigen Alkylrest mit 1—13 C-Atomen oder einen Pyridylrest darstellt,

oder ein Salz davon mittels enantiospezifische Dehydrogenaseaktivität aufweisenden, zu den Genera Streptomyces, Pseudomonas, Bacillus oder zur Coryneformgruppe gehörenden Mikroorganismen asymmetrisch zur $\alpha$-Ketocarbonsäure bzw. zu einem Salz davon dehydriert, aus einem erhaltenen Salz die Säure freisetzt und schliesslich das nicht dehydrierte Enantiomere der $\alpha$-Hydroxycarbonsäure und gegebenenfalls die $\alpha$-Ketocarbonsäure aus dem Reaktionsmedium isoliert.

2. Verfahren nach Anspruch 1, dadurch gekennzeichnet, dass man die erhaltene $\alpha$-Ketocarbonsäure in an sich bekannter Weise zur $\alpha$-Hydrocarbonsäure hydriert und diese als Ausgangsmaterial in den Prozess zurückführt.

3. Verfahren nach Anspruch 1 oder 2, dadurch gekennzeichnet, dass man als Ausgangssäure der Formel I 2-Hydroxypropionsäure, 2-Hydroxybuttersäure, 2-Hydroxyvaleriansäure, 2-Hydroxy-3-methylbuttersäure, 2-Hydroxycapronsäure, 2-Hydroxy-4-methylvaleriansäure, 2-Hydroxy-3,3-dimethylbuttersäure, 2-Hydroxyheptylsäure, 2-Hydroxy-4-methylcapronsäure, 2-Hydroxycaprylsäure, 2-Hydroxypelargonsäure, 2-Hydroxydecansäure, 2-Hydroxyundecansäure, 2-Hydroxydodecansäure, 2-Hydroxytridecansäure, 2-Hydroxytetradecansäure, 2-Hydroxypentadecansäure oder $\alpha$-Hydroxy-3-pyridylessigsäure verwendet.

4. Verfahren nach Anspruch 1, 2 oder 3, dadurch gekennzeichnet, dass man als Mikroorganismus den Stamm Bacillus megaterium NRRL B—11084; Bacillus sp. NRRL 11085; Bacillus freudenreichii ATCC 31301; einen der Coryneform-Gruppe angehörenden Stamm NRRL B—11086; ATCC 31300; NRRL B—11088; oder NRRL B—11087; Pseudomonas fluorescens NRRL 11089; Pseudomonas putida ATCC 31303 oder NRRL 11090; Pseudomonas sp. ATCC 31302, Streptomyces sp. NRRL 11083 oder NRRL 11091 verwendet.

## Revendications

1. Procédé pour la préparation d'acides $\alpha$-hydroxycarboxyliques optiquement actifs, caractérisé par le fait que l'on déshydrogène asymétriquement en l'acide $\alpha$-cétocarboxylique correspondant ou en un sel de celui-ci un acide D,L-$\alpha$-hydroxycarboxylique de formule générale:

R—CH—COOH I
|
OH

dans laquelle R représente un reste alkyle en $C_1$—$C_{13}$ à chaîne droite ou ramifiée ou un reste pyridyle, ou un sel de cet acide, au moyen de micro-organismes présentant une activité de déshydrase énantio-spécifique et appartenant aux genres Streptomyces, Pseudomonas ou Bacillus ou un groupe Coryne-form, on libère l'acide à partir d'un sel ainsi obtenu et, enfin, on isole du milieu de réaction l'énantiomère non déshydrogéné de l'acide $\alpha$-hydroxycarboxylique et éventuellement l'acide $\alpha$-cétocarboxylique.

2. Procédé suivant la revendication 1, caractérisé par le fait qu'on hydrogène, de manière connue en soi, l'acide $\alpha$-cétocarboxylique obtenu en acide $\alpha$-hydroxycarboxylique et que l'on recycle celui-ci comme matière première.

3. Procédé suivant la revendication 1 ou la revendication 2, caractérisé par le fait qu'on utilise, comme acides de formule I en tant que matière première, les acides 2-hydroxypropionique, 2-hydroxybutyrique, 2-hydroxyvalérianique, 2-hydroxy-3-méthylbutyrique, 2-hydroxycaproîque, 2-hydroxy-4-méthylvalérianique, 2-hydroxy-3,3-diméthylbutyrique, 2-hydroxyheptanoîque, 2-hydroxy-4-méthylcaproîque, 2-hydroxycaprylique, 2-hydroxypélargonique, 2-hydroxydécanoîque, 2-hydroxyundécanoîque, 2-hydroxy-dodécanoîque, 2-hydroxytridécanoîque, 2-hdyroxytétradécanoîque, 2-hydroxypentadécanoîque ou $\alpha$-hydroxy-3-pyridylacétique.

4. Procédé suivant l'une des revendications 1, 2 et 3, caractérisé par le fait qu'on utilise comme micro-organisme l'une des souches suivantes: Bacillus megaterium NRRL B—11084; Bacillus sp. NRRL 11085; Bacillus freudenreichii ATCC 31301; une souche NRRL B—11086; ATCC 31300; NRRL B—11088; ou NRRL B—11087 appartenant au groupe Coryneform; Pseudomonas fluorescens NRRL 11089; Pseudomonas putida ATCC 31303 ou NRRL 11090; Pseudomonas sp. ATCC 31302; Streptomyces sp. NRRL 11083 ou NRRL 11091.

**Claims**

1. Process for the manufacture of optically active $\alpha$-hydroxycarboxylic acids, characterized by asymmetrically dehydrogenating a D,L-$\alpha$-hydroxycarboxylic acid of the general formula

R—CH—COOH I
|
OH

wherein R represents a branched or straight-chain alkyl residue with 1—13 C-atoms or a pyridyl residue,
or a salt thereof to the $\alpha$-ketocarboxylic acid or to a salt thereof by means of microorganisms belonging to the genera Streptomyces, Pseudomonas, Bacillus or to the Coryneform group and having enantiospecific dehydrogenase activity, liberating the acid from a salt obtained and finally isolating the non-dehydrogenated enantiomer of the $\alpha$-hydroxycarboxylic acid and, if desired, isolating the $\alpha$-ketocarboxylic acid from the reaction medium.

2. Process according to claim 1, characterized in that the $\alpha$-ketocarboxylic acid obtained is hydrogenated in a manner known per se to the $\alpha$-hydroxycarboxylic acid and this is led back into the process as the starting material.

3. Process according to claim 1 or 2, characterized in that 2-hydroxypropionic acid, 2-hydroxybutyric acid, 2-hydroxyvaleric acid, 2-hydroxy-3-methylbutyric acid, 2-hydroxycaproic acid, 2-hydroxy-4-methylvaleric acid, 2-hydroxy-3,3-dimethylbutyric acid, 2-hydroxyheptylic acid, 2-hydroxy-4-methylcaproic acid, 2-hydroxycaprylic acid, 2-hydroxypelargonic acid, 2-hydroxydecanoic acid, 2-hydroxyundecanoic acid, 2-hydroxydodecanoic acid, 2-hydroxytridecanoic acid, 2-hydroxytetradecanoic acid, 2-hydroxypentadecanoic acid or $\alpha$-hydroxy-3-pyridylacetic acid is used as the starting acid of formula I.

4. Process according to claim 1, 2 or 3, characterized in that the strain Bacillus megaterium NRRL B—11084; Bacillus sp. NRRL 11085; Bacillus freudenreichii ATCC 31301; one of the strains NRRL B—11086; ATCC 31300; NRRL B—11088; or NRRL B—11087 belonging to the Coryneform group; Pseudomonas fluorescens NRRL 11089; Pseudomonas putida ATCC 31303 or NRRL 11090; Pseudomonas sp. ATCC 31302, Streptomyces sp. NRRL 11083 or NRRL 11091 is used as the microorganism.